# EUROPEAN PATENT APPLICATION

(11) **EP 3 040 706 A1**
(43) Date of publication of application: **06.07.2016**
(21) Application number: 15202957.5
(22) Date of filing: 29.12.2015
(51) Int. Cl.: G01N 21/3504, G01J 3/42, G01N 33/00, G01N 21/359

(54) **DETECTION OF HYDROCARBON GASES USING SHORT WAVE INFRARED TECHNOLOGY**

(30) Priority: 02.01.2015 US 201514588550
(71) Applicant: Sensors Unlimited, Inc., Princeton, NJ 08540 (US)
(72) Inventor: XIONG, Ziyou, Wethersfield, CT Connecticut 06109 (US); SAITTA, Salvatore, Willington, CT Connecticut 06279 (US); MANTESE, Joseph V., Ellington, CT Connecticut 06029 (US); ROZPLOCH, Robert, Newtown, PA Pennsylvania 18940 (US)
(74) Representative: Bridge, Kerry Ann

(57) **Abstract**

An apparatus for detecting a hydrocarbon gas, e.g. methane, propane, toluene, benzene, in a gas plume (24) includes a short wave infrared (SWIR) light source and an uncooled solid state SWIR sensor, e.g. an indium gallium arsenide sensor, in a camera (30) for imaging at least one absorption band of the hydrocarbon gas, e.g. in the range of 2.35 μm. Also included is a narrow bandpass filter (28), e.g. with a bandwidth from 2.0 to 2.5 μm, tuned to at least one narrow absorption band of the gas positioned in front of the sensor.

## Description

### BACKGROUND

The present invention relates to the detection of hydrocarbon gases, in particular the line of sight detection of methane.

The accumulation of undetected quantities of hydrocarbon gases may result in environmental and safety issues as well as suggested negative effects on global warming. Hydrocarbon gas detection may be performed by point detection methods utilizing detectors relying on the catalytic, electrochemical or thermal conductive properties of a subject gas. Other sensing techniques rely on the infrared properties of the gas, in particular, the absorption and emission spectrum of the gas.

Line of sight volume detection of a gas plume can both identify the location and the extent of the plume. Direct observation of a gas plume using solid state sensors are known in the art. Middle wavelength infrared (MWIR) radiation in the 3.2-3.4-micron wavelength range can be examined using indium antiminide (InSb) solid state detectors. Long wavelength infrared (LWIR) radiation in the about 7.7 micron wavelength range can be examined using mercury cadmium telluride (HgCdTe) solid state detectors. Both InSb and HgCdTe detector materials need to be cooled in order to operate. This feature adds cost to the systems. Other detectors that use active infrared illumination are known in the art. A low cost, portable, line of sight hydrocarbon gas detection device is needed.

### SUMMARY

An apparatus for detecting a hydrocarbon gas in a gas plume includes a short wave infrared (SWIR) light source and an uncooled solid state SWIR sensor in a camera for imaging at least one absorption band of the hydrocarbon gas. Also included is a narrow bandpass infrared filter tuned to at least one narrow SWIR absorption band of the gas positioned in front of the sensor. A particular gas of interest is methane.

In an embodiment, a method of detecting a hydrocarbon gas in a gas plume includes first irradiating the gas with short wave infrared (SWIR) light. Narrow SWIR absorption bands emanating from the gas that pass through a narrow band gap filter are then recorded by a camera containing an uncooled solid state indium gallium arsenide sensor. The narrow band gap filter is tuned to at least one narrow absorption band of the particular gas of interest.

### BRIEF DESCRIPTION OF THE DRAWINGS

FIG. 1 is a flow diagram illustrating a method of detection of a hydrocarbon gas according to an embodiment of the present invention.
FIG. 2 is a schematic diagram showing back illumination of a gas plume.
FIG. 3 is a plot showing the response of two sensors to bursts of methane leaks under back illumination.
FIG. 4 is a schematic diagram showing frontal illumination of a gas plume.

### DETAILED DESCRIPTION

Rapid detection of the position and extent of hydrocarbon gas plumes is a requirement in many industries including mining, energy production and distribution, and pollution control to name a few. Further requirements include portable, cost effective equipment and long range line of sight detection.

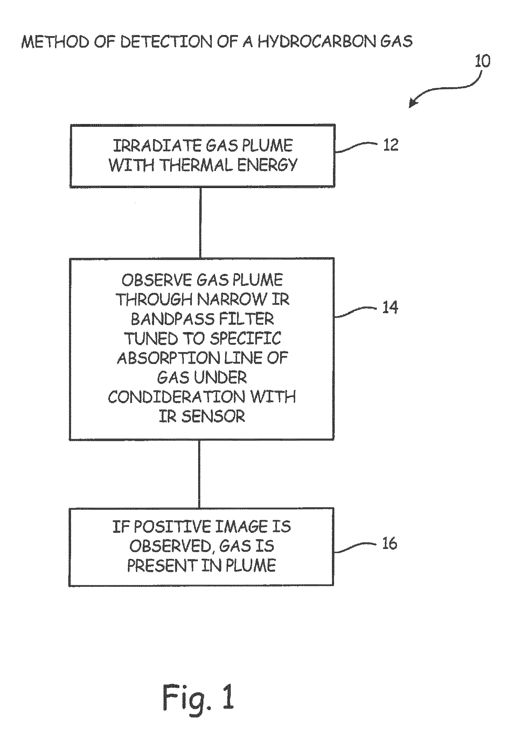

According to an embodiment of the present invention, a method of detection of a hydrocarbon gas plume is outlined in the flow diagram of FIG. 1. Detection method 10 comprises irradiating the gas plume with thermal energy to activate infrared signatures of gases in the plume (step 12). In the next step, the infrared signature of the plume is observed through a narrow infrared bandpass filter tuned to a specific absorption band of a hydrocarbon gas under consideration (step 14). This process is accomplished with an infrared sensor mounted in a suitable optical fixture such as a camera. If the gas under consideration is present in the plume, a sensor in the camera will detect the particular narrow wavelength of interest and register a positive signal (step 16).

The plume may be irradiated with thermal energy from a number of sources in step 12. A natural source is sunlight. Other sources may include incandescent sources such as search lights, spot lights and heat lamps. Lasers tuned to specific absorption bands of hydrocarbon gases may also be employed.

Line of sight detection of the infrared signature of a hydrocarbon gas plume differs from point detection methods that rely on analysis of gas samples collected at fixed sampling positions in the plume. Point detection sensors include electrochemical or thermal resistive chip type sensors and cavity enhanced laser absorption spectrometry sensors.

As noted earlier, solid state detection of long wavelength infrared (LWIR) signals from hydrocarbon gases around 7.7 microns can be detected using mercury cadmium telluride (HgCdTe) detectors. These detectors require additional cooling systems. In a similar manner, middle wavelength infrared (MWIR) signals can be detected by indium antimonide (InSb) detectors which also require additional cooling systems.

The present detection method relies on solid state short wavelength infrared (SWIR) detectors for hydrocarbon gas detection. This is advantageous because the present method uses solid state SWIR indium gallium arsenide (InGaAs) detectors that are uncooled, accurate and cost effective. A suitable SWIR sensing system for use in the present method is described in U.S. Pat. No. 6,555,890, which is incorporated by reference herein in its entirety.

The present method is able to detect the presence and extent of hydrocarbon gas plumes by examining infrared signatures of specific narrow absorption bands in the SWIR wavelength band of from 0.8 micron to 2.6 microns to detect specific hydrocarbon gases. Of particular interest in many embodiments is the detection of methane.

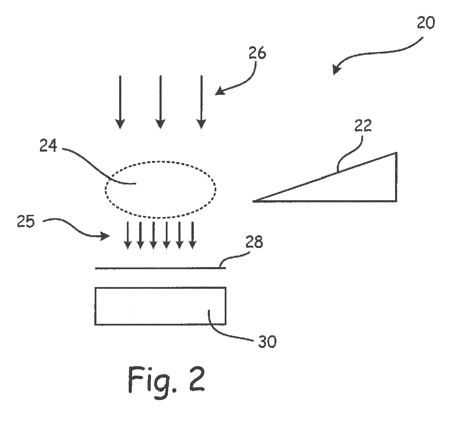

An exemplary demonstration of an embodiment of the invention is shown in FIG. 2. It is the purpose of the example to demonstrate the detection of methane. Example 20 includes methane nozzle 22 creating methane plume 24. Infrared radiation source 26 illuminates plume 24 with thermal radiation. In the example, radiation source 26 is a heat lamp. Under thermal illumination from radiation source 26, gas plume 24 absorbs and emits thermal infrared radiation 25 directed at narrow infrared bandpass filter 28 and infrared camera 30 containing InGaAs SWIR sensors (not shown).

Example 20 is an example of back illumination wherein plume 24 is located between radiation source 26 and narrow bandpass filter 28 and infrared camera 30. In the example, the 2.35 micron absorption band of methane was chosen as an indicator of methane detection. Filter 28 has a central wavelength of 2.25 microns and a bandpass region of 0.5 microns (that is, a wavelength range of 2.0 to 2.5 microns) to capture the 2.35 micron absorption band of methane.

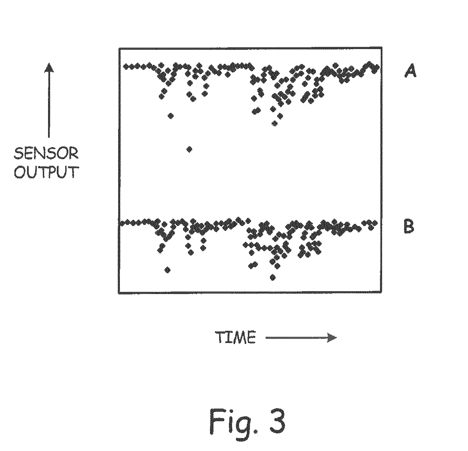

An experiment was performed wherein periodic bursts of methane were emitted from nozzle 22 while the gas plume was irradiated with thermal energy source 26. The results are shown in FIG. 3. FIG. 3 is a plot showing the outputs of two uncooled InGaAs SWIR sensors as a function of time during which short bursts of methane were introduced into the field of view of narrow bandpass filter 28 and infrared camera 30 (FIG. 2). There are simultaneous dips in the outputs of the sensing elements indicating rapid detection of the 2.35 micron absorption band of methane using the uncooled SWIR imaging technology. The decreased signal results from the methane blocking (absorbing) radiation 26 from reaching narrow bandpass filter 28 and infrared camera 30.

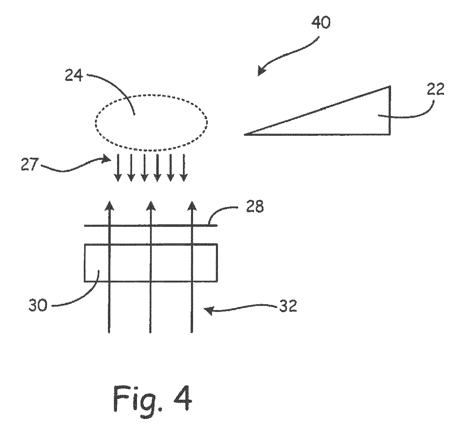

In an embodiment, gas plume 24 can be revealed using frontal illumination as shown schematically in FIG. 4. In this case, illumination source 32 is behind camera 30 and is directed in the same direction as the field of view (FOV) of camera 30. Directed infrared energy beams may be heat lamps, search lights, lasers or others known in the art. Solar energy is also a natural source of unfocused infrared illumination. When a hydrocarbon gas is illuminated by infrared radiation, the gas absorbs specific wavelengths according to the absorption spectrum of the gas. Some of the absorbed radiation is reflected, and reflected radiation in the wavelengths of interest is passed through bandpass filter 28 for detection by infrared camera 30. The observable distance to the plume is a function of sensor resolution which, in turn, depends on the detection optics, electronics and other variables.

Hydrocarbon gases of particular interest include acetone, benzene, methane, propyne and toluene.

### Discussion of Possible Embodiments

The following are non-exclusive descriptions of possible embodiments of the present invention.

An apparatus for detecting a hydrocarbon gas in a gas plume may include:
a shortwave infrared (SWIR) light source; an uncooled solid state SWIR sensor in a camera for imaging at least one absorption band of the hydrocarbon gas; and at least one narrow bandpass filter positioned in front of the sensor and tuned to the at least one narrow absorption band of the hydrocarbon gas.

The apparatus of the preceding paragraph can optionally include, additionally and/or alternatively any, one or more of the following features, configurations, and/or additional components:

The hydrocarbon gas may include acetone, benzene, methane, propyne and toluene.

The sensor may have a wavelength response of from 0.8 microns to 2.6 microns.

The sensor may be an indium gallium arsenide photodiode.

The SWIR light source may be incandescent, halogen, laser, or solar.

The filter bandpass range may be about 2.0 to 2.5 microns.

The presence of hydrocarbon gas may be confirmed by detection of absorption or reflection of radiation by the plume at a wavelength of about 2.35 microns.

A method of detecting a hydrocarbon in a gas plume may include: irradiating the gas plume with shortwave infrared (SWIR) light; positioning a camera containing an uncooled indium gallium arsenide SWIR sensor pointed at the gas plume; positioning at least one narrow bandpass filter tuned to at least one SWIR absorption band of the gas in front of the camera; and detecting at least one SWIR absorption band of the gas with the sensor.

The method of the preceding paragraph can optionally include additionally and/or alternatively any, one or more of the following features, configurations and/or additional components:

The hydrocarbon gas may include acetone, benzene, methane, propyne and toluene.

They hydrocarbon gas may be methane.

The SWIR light source may be incandescent, halogen, laser, or solar.

The narrow bandpass filter may have a bandpass region tuned to about 2.35 microns.

The sensor may be an indium gallium arsenide photodiode.

The sensor may have a wavelength response of from 0.5 microns to 2.6 microns.

Although the present invention has been described with reference to preferred embodiments, workers skilled in the art will recognize that changes may be made in form and detail without departing from the spirit and scope of the invention.

## Claims

1. An apparatus for detecting a hydrocarbon gas in a gas plume (24), the apparatus comprising:
a short wave infrared (SWIR) light source;
an uncooled solid state SWIR sensor in a camera (30) for imaging at least one absorption band of the hydrocarbon gas; and
at least one narrow bandpass filter (28) positioned in front of the sensor and tuned to the at least one narrow absorption band of the hydrocarbon gas.

2. The apparatus of claim 1 wherein the hydrocarbon gas is selected from the group consisting of acetone, benzene, methane, propyne, and toluene.

3. The apparatus of claim 1 or 2 wherein the hydrocarbon gas is methane.

4. The apparatus of any preceding claim wherein the sensor has a wavelength response of from 0.8 microns to 2.6 microns.

5. The apparatus of any preceding claim wherein the sensor is an indium gallium arsenide photodiode.

6. The apparatus of any preceding claim wherein the SWIR light source is incandescent, halogen, laser, or solar.

7. The apparatus of any preceding claim wherein the filter bandpass range is about 2.0 to 2.5 microns.

8. The apparatus of any preceding claim where presence of the hydrocarbon gas is confirmed by detection of absorption or reflection of radiation by the plume at a wavelength of about 2.35 microns.

9. A method of detecting a hydrocarbon in a gas plume (24) comprising:
irradiating the gas plume with short wave infrared (SWIR) light;
positioning a camera (30) containing an uncooled indium gallium arsenide SWIR sensor pointed at the gas plume;
positioning at least one narrow bandpass filter (28) tuned to at least one SWIR absorption band of the gas in front of the camera (30); and detecting at least one SWIR absorption band of the gas with the sensor.

10. The method of claim 9 wherein the hydrocarbon gas is selected from the group consisting of acetone, benzene, methane, propyne, and toluene.

11. The method of claim 9 or 10 wherein the hydrocarbon gas is methane.

12. The method of any of claims 9 to 11 wherein the SWIR light source is incandescent, halogen, laser, or solar.

13. The method of any of claims 9 to 12 wherein the narrow bandpass filter has a bandpass region tuned to about 2.35 microns.

14. The method of any of claims 9 to 13 wherein the sensor is an indium gallium arsenide photodiode.

15. The method of any of claims 9 to 14 wherein the sensor has a wavelength response of from 0.5 microns to 2.6 microns.
